(19) Europäisches Patentamt

European Patent Office

Office européen des brevets

(11) **EP 1 044 695 B1**

(12) **EUROPÄISCHE PATENTSCHRIFT**

(45) Veröffentlichungstag und Bekanntmachung des Hinweises auf die Patenterteilung:
**05.04.2006 Patentblatt 2006/14**

(51) Int Cl.:
*A61M 1/16* *(2006.01)*          *A61M 1/34* *(2006.01)*
*A61M 1/36* *(2006.01)*

(21) Anmeldenummer: **00108111.6**

(22) Anmeldetag: **13.04.2000**

(54) **Vorrichtung zur Ermittlung des Blutflusses in einem Gefäßzugang**

Device for determination of blood flow in a vessel access

Appareil pour la détermination du flux sanguin dans un site d'accès à un vaisseau

(84) Benannte Vertragsstaaten:
**DE ES FR GB IT**

(30) Priorität: **16.04.1999 DE 19917197**

(43) Veröffentlichungstag der Anmeldung:
**18.10.2000 Patentblatt 2000/42**

(73) Patentinhaber: **Fresenius Medical Care Deutschland GmbH**
**61352 Bad Homburg v.d.H. (DE)**

(72) Erfinder: **Kleinekofort, Wolfgang**
**65779 Kelkheim (DE)**

(74) Vertreter: **Luderschmidt, Schüler & Partner GbR Patentanwälte,**
**John-F.-Kennedy-Strasse 4**
**65189 Wiesbaden (DE)**

(56) Entgegenhaltungen:
**WO-A-97/10013**          **DE-A- 4 024 434**
**DE-C- 19 541 783**       **US-A- 5 830 365**

**Beschreibung**

Die Erfindung betrifft eine Vorrichtung zur Ermittlung des Blutflusses in einem Gefäßzugang für eine extrakorporale Blutbehandlung

Bei Verfahren der chronischen Blutreinigungstherapie wie Hämodialyse, Hämofiltration und Hämodiafiltration wird Blut über einen extrakorporalen Kreislauf durch eine Blutbehandlungseinheit, beispielsweise einen Dialysator oder Filter geleitet. Als Zugang zum Blutgefäßsystem wird häufig operativ eine arteriovenöse Fistel angelegt, die im allgemeinen mit einer arteriellen und venösen Kanüle punktiert wird (Doppelnadel-Dialyse). Ebenso ist der Einsatz eines Gefäßimplantats (PTFE graft) möglich. Wenn nachfolgend von dem Begriff "Gefäßzugang" die Rede ist, wird darunter jede Art eines Zugangs zu einem Blutgefäß eines Patienten, insbesondere aber die Verbindung zwischen einer Arterie und einer Vene des Patienten verstanden.

Typische Flüsse innerhalb eines einwandfrei funktionierenden Gefäßzugangs liegen im Bereich von 1100 ml/min. Die Messung von Blutfluß und Gefäßdruckzustand ist für die Überwachung der Funktion von entscheidender Bedeutung. Gefäßimplantate, die einen Fluß unterhalb von 600 bis 800 ml/min sowie abnorme Drücke aufweisen, zeigen eine deutlich erhöhtes Thromboserisiko. Thrombosen entwickeln sich infolge unerkannter Stenosen, die zu einer Verminderung des Blutflusses im Gefäßzugang führen. Durch die Früherkennung von Gefäßzugängen mit abnehmendem Blutfluß ist es daher möglich, bevorstehende Thrombosen zu vermeiden. Weiterhin kann durch die Identifizierung von Gefäßzugängen mit pathologisch hohen Flüssen oberhalb 2000 ml/min einer Überbelastung des Herz-Kreislaufsystems des Patienten vorgebeugt werden.

Die DE 40 24 434 A1 beschreibt eine Vorrichtung zur Ultrafiltrationskontrolle bei Blutreinigungsverfahren, die über eine im extrakorporalen Blutkreislauf angeordnete Druckmeßeinrichtung sowie eine Auswerteinheit verfügt, in der die gemessenen Druckwerte in zeitlicher Abfolge gespeichert und aus der Änderung der Druckwerte auf eine Veränderung der Blutviskosität geschlossen wird. Weiterhin wird der Fisteldruck ermittelt.

Eine Vorrichtung zur Messung des Fistelflusses ist aus der DE 195 41 783 C1 bekannt. Die Messung des Fistelflusses beruht darauf, daß die Temperatur im arteriellen Zweig des extrakorporalen Kreislaufs unter Variation des extrakorporalen Blutflusses gemessen wird.

Ein weiteres Verfahren zur Ermittlung des Blutflusses im Gefäßzugang beruht auf der Messung der Rezirkulation vor und nach Vertauschen des arteriellen und venösen Blutschlauches an den Kanülen. Diese Methode zeigt gute klinische Ergebnisse. Nachteilig ist jedoch, daß bei unsachgemäßem Vertauschen der Schläuche die Gefahr von Blutverlusten, Infektionen sowie ein Restrisiko von Luftembolien besteht.

Im klinischen Alltag wird der statische Druck im Gefäßzugang nach Abschalten der Blutpumpe sowie der Ultrafiltration gemessen. Wenn aber die Blutpumpe angehalten wird, besteht das Risiko der Koagulation im Blutschlauchsystem.

Der Erfindung liegt die Aufgabe zugrunde, eine technisch relativ einfach zu realisierende Vorrichtung bereitzustellen, mit der sich der Blutfluß im Gefäßzugang mit hoher Sicherheit ermitteln läßt, ohne daß ein Vertauschen der Blutschlauchanschlüsse notwendig ist.

Die Lösung dieser Aufgabe erfolgt mit der Vorrichtung des Anspruchs 1.

Die beanspruchte Vorrichtung verfügt zum Messen des Drucks im arteriellen und/oder venösen Zweig über eine arterielle und/oder venöse Druckmeßeinrichtung. Eine Steuereinheit ist vorgesehen, um durch Verändem der Flußrate der im extrakorporalen Kreislauf angeordneten Blutpumpe den extrakorporalen Blutfluß variieren zu können. Die Meßwerte werden in einer Speichereinheit gespeichert. Der Blutfluß im Gefäßzugang wird aus den gespeicherten Meßwerten in einer Recheneinheit berechnet.

Nachfolgend werden unter Bezugnahme auf die Zeichnungen verschiedene Ausführungsbeispiele der Erfindung näher erläutert.

Es zeigen:

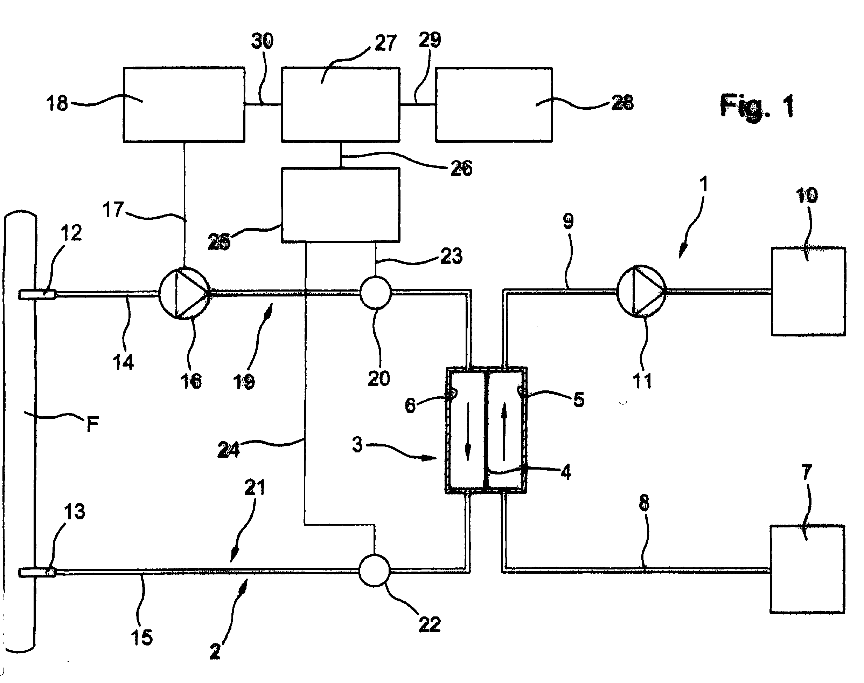

Figur 1   eine Vorrichtung zur Ermittlung des Blutflusses und des statischen Drucks im Gefäßzugang zusammen mit einer Dialysevorrichtung in einer stark vereinfachten schematischen Darstellung,

Figur 2   die Druck- bzw. Flußänderung beim Unterbrechen des Gefäßzugangs zwischen arteriellem und venösem Anschluß, wenn der Blutfluß im Gefäßzugang größer als der Blutfluß im extrakorporalen Kreislauf ist,

Figur 3   den Druck im arteriellen bzw. venösen Zweig des extrakorporalen Kreislaufs bei offenem und unterbrochenem Gefäßzugang als Funktion des extrakorporalen Blutflusses für eine simulierte Dialysebehandlung,

Figur 4   die Differenz zwischen dem arteriellen bzw. venösen Druck bei unterbrochenem Gefäßzugang und dem arteriellen bzw. venösen Druck bei offenem Gefäßzugang als Funktion des extrakorporalen Blutflusses für eine simulierte Dialysebehandlung und

Figur 5      den arteriellen bzw. venösen Druck als Funktion des extrakorporalen Blutflusses für eine simulierte Dialysebehandlung.

Die Vorrichtung zur Ermittlung des Blutflusses $Q_F$ im Gefäßzugang (Fistelfluß) kann eine separate Baugruppe bilden. Sie kann aber auch Bestandteil der Blutbehandlungsvorrichtung sein, zumal einige ihrer Komponenten in den bekannten Blutbehandlungsvorrichtungen bereits vorhanden sind. Nachfolgend wird die Vorrichtung zur Ermittlung des Fistelflusses zusammen mit den wesentlichen Komponenten der Blutbehandlungsvorrichtung beschrieben. Bei der Blutbehandlungsvorrichtung handelt es sich in dem vorliegenden Ausführungsbeispiel um ein konventionelles Dialysegerät.

Das Dialysegerät umfaßt einen Dialysierflüssigkeitskreislauf 1 und einen extrakorporalen Blutkreislauf 2, zwischen denen ein Dialysator 3 angeordnet ist, der durch eine semipermeable Membran 4 in eine Dialysierflüssigkeitskammer 5 und eine Blutkammer 6 getrennt ist. Von einer Dialysierflüssigkeitsquelle 7 führt eine Dialysierflüssigkeitszuführleitung 8 zu dem Einlaß der Dialysierflüssigkeitskammer, von deren Auslaß eine Dialysierflüssigkeitsabführleitung 9 zu einem Abfluß 10 führt. In die Dialysierflüssigkeitsabführleitung ist zur Förderung der Dialysierflüssigkeit eine Dialysierflüssigkeitspumpe 11 geschaltet.

Die Fistel F des Patienten ist mit einer arteriellen und venösen Kanüle 12, 13 punktiert. Von dem arteriellen Anschluß führt eine Blutzuführleitung 14 zu dem Einlaß der Blutkammer, während eine Blutabführleitung 15 von deren Auslaß zu dem venösen Anschluß führt. In die Blutzufuhrleitung ist eine den Blutfluß im extrakorporalen Kreislauf vorgebende Blutpumpe 16 geschaltet, die über eine Steuerleitung 17 mit einer Steuereinheit verbunden ist. Mit der Steuereinheit 18 kann die Förderrate der Blutpumpe innerhalb eines vorgegebenen Bereichs verändert werden. Zum Messen des Drucks im arteriellen Zweig 19 ist eine arterielle Druckmeßeinrichtung 20 und zum Messen des Drucks im venösen Zweig 21 des extrakorporalen Kreislaufs ist eine venöse Druckmeßeinrichtung 22 vorgesehen. Die beiden Druckmeßeinrichtungen sind über Datenleitungen 23, 24 mit einer Speichereinheit 25 verbunden, in der die gemessenen Werte in zeitlicher Abfolge digital gespeichert werden. Die Speichereinheit ist über eine Datenleitung 26 mit einer Recheneinheit 27 verbunden, die aus den Meßwerten den Fistelfluß und den statischen Druck in der Fistel berechnet. Die ermittelten Größen werden auf einer Anzeigeeinheit 28 angezeigt, die über eine Datenleitung 29 mit der Recheneinheit verbunden ist. Zur Steuerung des Programmablaufs ist die Recheneinheit ihrerseits über eine Datenleitung 30 mit der Steuereinheit verbunden. Die Recheneinheit kann ein konventioneller Mikroprozessor sein.

Im folgenden wird das Prinzip der Messung im einzelnen erläutert.

Während der laufenden Dialysebehandlung werden der arterielle und venöse Druck $p_{art}$, $p_{ven}$ im extrakorporalen Kreislauf als Funktion des extrakorporalen Blutflusses $Q_B$ aufgenommen. Aus der Messung erhält man folgende Funk-tionen:

$p_{art}$ ($Q_B$):      Arterieller Druck im extrakorporalen Kreislauf als Funktion von $Q_B$.

$p_{ven}$ ($Q_B$):      Venöser Druck im extrakorporalen Kreislauf als Funktion von $Q_B$.

Nach Beendigung der Messung wird der Gefäßzugang zwischen arterieller und venöser Kanüle abgedrückt und der veränderte extrakorporale Druck als Funktion des extrakorporalen Blutflusses aufgezeichnet. Die Kompression des Gefäßzugangs zwischen den Kanülen entweder mit den Fingern oder einem Kompressionsschlauch stellt eine bereits klinisch etablierte Methode zur Bestimmung des Gefäßwiderstandes dar. Weiterhin konnte bereits gezeigt werden, dass sich das Herzminutenvolumen durch kurzzeitiges Abdrücken des Gefäßzugangs ($t \leq 2$ min) nicht ändert. Um artifizielle Druckschwankungen zu vermeiden, sollte der Patient während der Messungen seine Position beibehalten. Zusätzlich wird die Ultrafiltrationsrate nicht verändert. Somit kann während der Messung von hämodynamischer Stabilität ausgegangen werden. Man erhält:

$p_{art\ komp}$ ($Q_B$):      Arterieller Druck im extrakorporalen Kreislauf als Funktion von $Q_B$ nach Abdrücken des Gefäßzugangs.

$p_{ven\ komp}$ ($Q_B$):      Venöser Druck im extrakorporalen Kreislauf als Funktion von $Q_B$ nach Abdrücken des Gefäßzugangs.

Die bei laufender Blutpumpe im extrakorporalen Kreislauf gemessenen Drücke setzen sich zusammen aus dem dynamischen Druck im Extrakorporalsystem sowie dem dynamischen Druck im Gefäßzugang des Patienten. Der dynamische Druck im Extrakorporalsystem ist eine Funktion des extrakorporalen Blutflusses, der Blutviskosität sowie die Summe der Flusswiderstände im extrakorporalen Kreislauf. Der dynamische Druck im Gefäßzugang des Patienten ist eine Funktion des systemischen Blutdrucks sowie der systemischen vaskulären Flusswiderstände. Der Fisteldruck ist somit ein patientenspezifischer Parameter, er hängt zusätzlich ab von der Art des Gefäßzugangs, der Viskosität des Blutes sowie vom Gefäßsystem, das den Gefäßzugang mit Blut versorgt. Analog zum dynamischen Druck im Extrakorporalsystem führt eine Änderung des Fisteldrucks, z. B. durch Blutdruckschwankung, Viskositäterhöhung oder Lageänderung des Patienten zur Änderung sowohl des arteriellen als auch des venösen extrakorporalen Druckwertes.

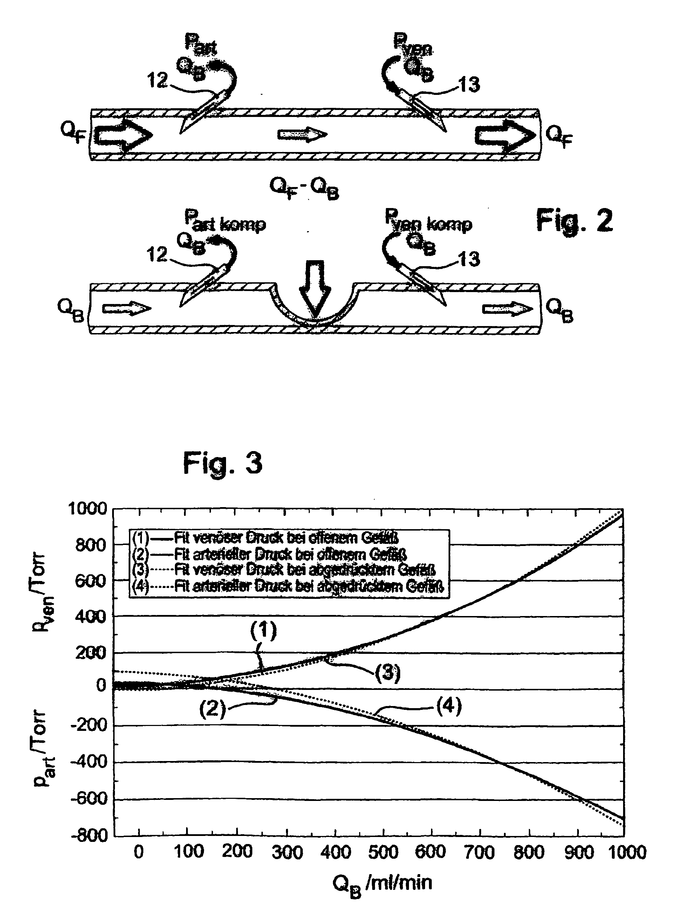

Figur 2 zeigt schematisch die Fluss- und Druckverhältnisse vor bzw. nach Abdrücken der Fistel. Bei stehender Blutpumpe und abgedrücktem Gefäßzugang liegt an der arteriellen Kanüle 12 der systemische arterielle Blutdruck des Patienten an. Dieser liegt je nach Blutdruck im Bereich von 6666,10 - 19.998,30 Pa (50-150 Torr). Der Druck an der venösen Kanüle 13 entspricht dem venösen Rücklaufdruck im Gefäßzugang des Patienten (399,97 -1999,83 Pa (3-15 Torr)). Bei offenem Gefäßzugang beträgt der arterielle Druck im Gefäßzugang bei intakten Fisteln ca. 3599,69 Pa (27 Torr) und bei intakten PTFE grafts ca. 6532,78 Pa (49 Torr). Der venöse Druck liegt bei ca. 2266,47 Pa (17 Torr) (Fistel) bzw. ca. 4666,27 Pa (35 Torr) (graft).

Bei laufender Blutpumpe und offenem Gefäßzugang ist der Fistelfluss $Q_F$ in der Regel größer als der extrakorporale Blutfluss $Q_B$. In diesem Fall fließt während der Dialysebehandlung zwischen den Kanülen innerhalb des Gefäßes ein reduzierter Fistelfluss $Q_F$ - $Q_B$. Für den Fall, dass der extrakorporale Blutfluss größer als der Fluss im Gefäß ist ($Q_B > Q_F$), wird die Differenz $Q_F$ - $Q_B$ negativ, d. h. es tritt ein Rezirkulationsfluss von der venösen zur arteriellen Kanüle auf. Im Fall, dass der extrakorporale Blutfluss genau gleich dem Fluss im Gefäßzugang ist, fließt zwischen arterieller und venöser Kanüle kein Blut durch den Gefäßzugang. Im folgenden wird zwischen den drei Fällen $Q_B<Q_F$, $Q_B>Q_F$ und $Q_B = Q_F$ unterschieden.

$Q_B<Q_F$: Im Fall, dass der reduzierte Fluss $Q_F$ - $Q_B$ zwischen arterieller und venöser Kanüle durch Abdrücken behindert wird, bildet sich an der arteriellen Nadel ein Staudruck aus. Dadurch erhöht sich der arterielle extrakorporale Druck $p_{art. komp}$. Hierbei gilt: Je höher der reduzierte Fluss $Q_F$-$Q_B$, desto höher ist der Staudruck. Hingegen sinkt der venöse extrakorporale Druck $p_{ven komp}$ ab, wobei der venöse Druckabfall ebenfalls abhängig vom reduzierten Fluss $Q_F$-$Q_B$ ist.

$Q_B=Q_F$: In diesem Grenzfall tritt beim Abdrücken des Gefäßes zwischen den Kanülen keine Änderung der Druck- und Flussverhältnisse ein.

$Q_B>Q_F$: Wenn der extrakorporale Blutfluss größer als der Fluss im Gefäßzugang ist, tritt Rezirkulation auf. Der Rezirkulationsfluss von venöser zu arterieller Kanüle wird durch Kompression des Gefäßes zwischen den Kanülen ausgeschaltet. Dadurch sinkt der arterielle extrakorporale Druck, wobei die resultierende (negative) Druckdifferenz vom Rezirkulationsfluss abhängig ist. Der venöse extrakorporale Druck steigt hingegen leicht an, da das Abfließen des venösen Blutes durch Unterbindung des Rezirkulationsflusses behindert wird.

Die folgende Tabelle faßt die arteriellen und venösen Druckänderungen für die oben aufgeführten Flußverhältnisse zusammen.

| $\Delta p$ art. (QB) | $\Delta p$ ven. (QB) | Fluß im Gefäßzugang |
|---|---|---|
| + | - | $Q_B<Q_F$ |
| 0 | 0 | $Q_B=Q_F$ |
| - | + | $Q_B>Q_F$ |

Hierbei ist:

$$\Delta p_{art}(Q_B) = p_{art\ komp}(Q_B) - p_{art}(Q_B) \qquad \text{(Gleichung 1)}$$

$$\Delta p_{ven}(Q_B) = p_{ven\ komp}(Q_B) - p_{ven}(Q_B) \qquad \text{(Gleichung 2)}$$

Es zeigt sich, daß bereits durch eine einzige Differenzdruckmessung bei laufender Behandlung eine qualitative Aussage über den Fluß im Gefäßzugang möglich ist.

Figur 3 zeigt für eine simulierte Dialysebehandlung die Druck-Fluß-Kurve bei offenem und abgedrücktem Gefäßzugang bei einem Fistelfluß von $700\pm5$ ml/min. Es zeigt sich, daß der Fluß im extrakorporalen Kreislauf turbulent ist. Folglich verläuft die Funktion $p=f(Q_B)$ nichtlinear, kann jedoch mit quadratischen Polynomen vom Typ $y=a+bx+cx^2$ mit hoher Korrelation angepaßt werden.

Zur Messung der Funktion $p=f(Q_B)$ wurde der Blutfluß im Bereich von 50-550 ml/min variiert und der zugehörige extrakorporale Druck ausgelesen. Anschließend wurden die Funktionen mit Polynomen zweiten Grades angepaßt und extrapoliert. Die Korrelationskoeffizienten lagen im Bereich von $R^2>0,998$.

Bei stehender Blutpumpe beträgt der arterielle Druck im offenen Gefäßzugang ca. 4532,95 Pa (34 Torr), der venöse Druck liegt im Bereich von 4266,30 Pa (32 Torr). Durch Kompression des Gefäßes zwischen der Kanülen erhöht sich der statische arterielle Druck auf ca. 12.532,27 Pa (94 Torr). Dieser Wert entspricht somit dem mittleren systemischen Druck des arteriellen Systems. Der statische venöse Druck sinkt auf ca. 933, 25 Pa (7 Torr) und spiegelt den venösen Rücklaufdruck wieder. Bei Erhöhung des Blutflusses sinkt die anfängliche Druckdifferenz zwischen komprimiertem und offenem Gefäß, da die abgedrückte Stelle des Gefäßes durch den extrakorporalen Kreislauf zunehmend überbrückt wird.

Im Schnittpunkt der jeweiligen arteriellen und venösen Funktionspaare $p=f(Q_B)$ gilt $\Delta p=0$. Im Falle, dass sich der extrakorporale Druck bei Kompression des Gefäßes nicht ändert, muss folglich der resultierende Fluss zwischen den Kanülen bei offenem Gefäß null sein, d. h. Fistelfluss und extrakorporaler Blutfluss sind identisch. Somit kann der Fistelfluss direkt aus dem zum Schnittpunkt zugehörigen $Q_B$-Wert abgelesen werden.

Die Berechnung der Schnittpunkte der Polynomfunktionen zweiten Grades erfolgt nach folgendem Verfahren:

Das Polynom zweiten Grades für die Druck-Fluss-Kurve bei offenem Gefäß sei gegeben durch:

$$y_1=a_1 + b_1x +c_1x^2 \qquad\qquad \text{(Gleichung 3)}$$

Bei abgedrücktem Gefäß sei die Polynomfunktion gegeben durch:

$$y_2=a_2+b_2x+c_2x^2 \qquad\qquad \text{(Gleichung 4)}$$

Gleichsetzen von Gleichung 3 und Gleichung 4 liefert:

$$a_1+b_1x+c_1x^2 = a_2+b_2x+c_2x^2 \qquad\qquad \text{(Gleichung 5)}$$

Daraus folgt nach Umformung:

$$(a_1-a_2) + (b_1-b_2)x + (c_1-c_2)x^2=0 \qquad\qquad \text{(Gleichung 6)}$$

Mit folgenden Substitutionen

$$a_1-a_2=A$$

$$b_1-b_2=B$$

$$c_1-c_2=C$$

folgt für die Lösung der gemischtquadratischen Gleichung 6:

$$x_1 = \frac{-B+\sqrt{B^2-4AC}}{2C} \qquad\qquad \text{(Gleichung 7)}$$

$$x_2 = \frac{-B - \sqrt{B^2 - 4AC}}{2C}$$

(Gleichung 8)

Gleichung 6 hat zwei Lösungen, die entweder verschiedenen reell, gleich reell oder konjugiert komplex sein können. Die Entscheidung darüber liefert die Diskriminante D:

$$D = B^2 - 4AC$$

(Gleichung 9)

Ist D positiv, so gibt es zwei verschiedene reelle Lösungen. Ist D=0, so gibt es eine reelle Doppellösung. Ist dagegen D negativ, so hat Gleichung 4 zwei konjugiert komplexe Lösungen. Im vorliegenden Fall ist D immer positiv, Gleichung 4 liefert folglich zwei verschieden reelle Schnittpunkte. Von 21 physikalischem Interesse sind lediglich Werte x>0 (positiver extrakorporaler Blutfluß). Somit ist der gesuchte Schnittpunkt der Druck-Fluß-Kurven die positive Lösung aus Gleichung 7 und 8.

Die folgende Tabelle faßt die Konstanten der Anpassungsrechnungen vom Typ $y=a+bx+cx^2$ der Druck-Fluß-Kurve $p=f(Q_B)$ aus Figur 3 zusammen.

| Druck-Fluß-Kurve | a | b | c | $R^2$ |
|---|---|---|---|---|
| Arteriell bei offenem Gefäß | 30,0714 | -0,07591 | $-6,66965 \cdot 10^{-4}$ | 0,99915 |
| Arteriell bei abgedrücktem Gefäß | 88,40084 | -0,13109 | $-7,050 \cdot 10^{-4}$ | 0,99970 |
| Venös bei offenem Gefäß | 19,45073 | 0,08208 | $8,70911 \cdot 10^{-4}$ | 0,99988 |
| Venös bei abgedrücktem Gefäß | 3,11153 | 0,0633 | $9,45423 \cdot 10^{-5}$ | 0,99994 |

Einsetzen der jeweiligen Werte in Gleichung 6 und Berechnung nach Gleichung 7 und 8 liefert die sich aus folgender Tabelle ergebenden Schnittpunkte:

| Wertepaar | Schnittpunkt nach Gleichung 7 | Schnittpunkt nach Gleichung 8 |
|---|---|---|
| Arterielle Druck-Fluß-Kurve | 723 ml/min | -2284 ml/min |
| Venöse Druck-Fluß-Kurve | -439 ml/min | 691 ml/min |

Der Fistelfluß wird durch Bildung des Mittelwertes berechnet. Im Bereich positiver Flüsse beträgt der Mittelwert des errechneten Flusses im Gefäßzugang 707 $\pm$ 23 ml/min.

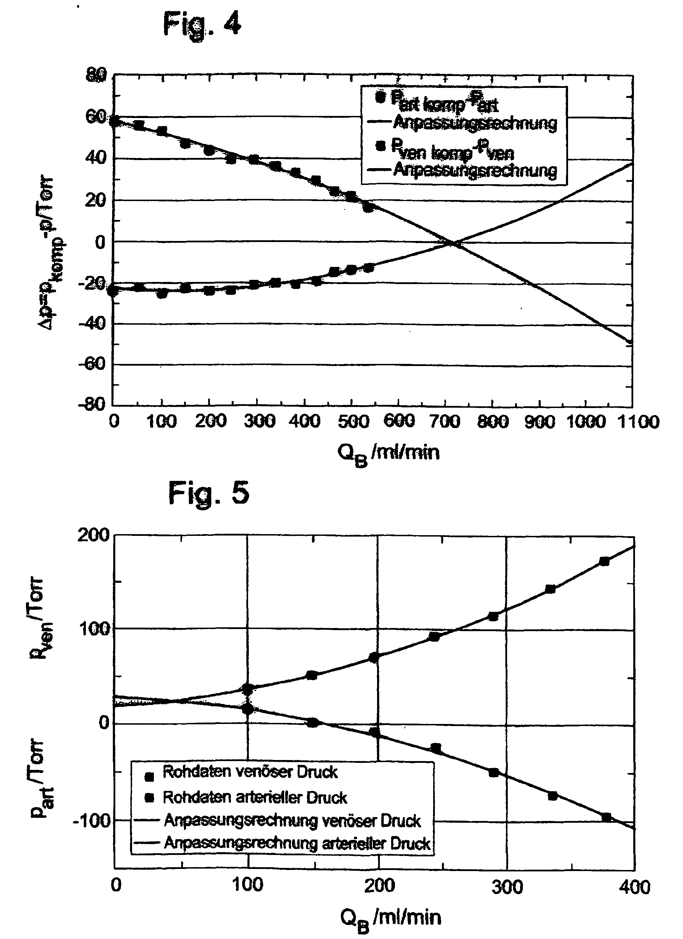

Eine alternative Darstellung der Druckmessung bei offenem und abgedrücktem Gefäßzugang zeigt Figur 4. Hier ist die Druckdifferenz $\Delta p$ nach Gleichung 1 und 2 als Funktion des effektiven extrakorporalen Blutflusses aufgetragen. Die Meßdaten wurden wiederum mit Polynomen zweiten Grades angepaßt. Im Fall, daß der extrakorporale Druck bei offener und bei abgedrückter Fistel gleich ist ($\Delta p=0$), sind extrakorporaler Blutfluß und Fluß im Gefäßzugang identisch. Folglich kann sowohl aus dem gemeinsamen Schnittpunkt der Polynome als auch aus den Schnittpunkten der einzelnen Polynome mit der x-Achse der Fistelfluß bestimmt werden. Die folgende Tabelle faßt die Konstanten der Anpassungsrechnung vom Typ $y=a+bx+cx^2$ aus Figur 4 zusammen.

| Druckdiffenenz | a | b | c | $R^2$ |
|---|---|---|---|---|
| $\Delta p_{art}(Q_B)$ nach Gleichung 1 | 58,24088 | -0,05247 | $-4,04643 \cdot 10^{-5}$ | 0,98834 |
| $\Delta p_{ven}(Q_B)$ nach Gleichung 2 | -22,90486 | -0,0149 | $6,44045 \cdot 10^{-5}$ | 0,9222 |

Die Schnittpunktberechnung der in Figur 4 dargestellten Polynome nach Gleichung 7 und 8 liefert als Flußwerte $Q_F = 719$ ml/min. Die Schnittpunkte der Polynome mit der x-Achse können berechnet werden, indem der y-Wert gleich null gesetzt wird:

$$a+bx+cx^2 = 0 \hspace{4cm} \text{(Gleichung 10)}$$

Analog zu Gleichung 6 wird die Lösung der gemischtquadratischen Gleichung 10 somit aus den Gleichungen 7 und 8 berechnet. Die jeweiligen $Q_F$-Werte betragen 715 ml/min (arterielle Kurve in Figur 4) bzw. 723 ml/min (venöse Kurve in Figur 4).

Bei dem Verfahren der Fistelflussmessung werden während der laufenden Dialysebehandlung der arterielle und venöse Druck im extrakorporalen Kreislauf als Funktion des extrakorporalen Blutflusses $Q_B$ aufgenommen.

Der statistische arterielle und venöse Druck kann wie folgt ermittelt werden: Die Funktion $p_{art}$ ($Q_B$) und $p_{ven}$ ($Q_B$) werden mit quadratischen Polynomen vom Typ $y=a+bx+cx^2$ angepasst. Anschließend wird der y-Achsenabschnitt a von arterieller und venöser Druck-Fluss-Kurve errechnet. An diesem Punkt der Kurven ist der extrakorporale Druck gleich dem statischen Druck im Gefäßzugang plus dem hydrostatischen Druck, der durch Höhenunterschiede zwischen extrakorporalem Drucksensor und Gefäßzugang zustande kommt. In guter Näherung kann man pro cm Höhendifferenz ca. 102,66 Pa (0,77 Torr) Druckdifferenz annehmen.

Figur 5 zeigt den Funktionswert von arterieller und venöser Druck-Fluss-Kurve $p=f(Q_B)$ für eine simulierte Dialysebehandlung sowie die zugehörige mathematische Anpassung. Die Funktion $p=f(Q_B)$ ist zwar nichtlinear, sie kann jedoch mit quadratischen Polynomen vom Typ $y=a+bx+cx^2$ mit hoher Korrelation angepasst werden. Durch Einsetzen von x=0 als Bedingung erhält man y=a, d. h. der Schnittpunkt des Polynoms mit der y-Achse ist durch die Polynomkonstante a festgelegt. Es ergeben sich die aus der folgenden Tabelle ersichtlichen Parameter:

| Druck-Fluss-Kurve | a | b | c | $R^2$ |
|---|---|---|---|---|
| Arterieller extrakorporaler Druck | 30,0714 | -0,07591 | $-6,66965 \cdot 10^{-4}$ | 0,99915 |
| Venöser extrakorporaler Druck | 19,45073 | 0,08208 | $8,709711 \cdot 10^{-4}$ | 0,999888 |

Die Vorrichtung zur Ermittlung des Fistelflusses und des statischen arteriellen und venösen Drucks arbeitet nun wie folgt:

Während der Dialysebehandlung leitet die Steuereinheit 18 den Meßvorgang dadurch ein, daß der Blutfluß $Q_B$ durch Veränderung der Flußrate der Blutpumpe 16 ausgehend von einem unteren Grenzwert kontinuierlich innerhalb eines vorgegebenen Bereichs bis zu einem oberen Grenzwert erhöht wird. Dabei wird der arterielle und venöse Druck $p_{art}$, $p_{ven}$ von der arteriellen bzw. venösen Druckmeßeinrichtung 20, 22 gemessen. Die Meßwerte werden in der Speichereinheit 25 gespeichert. Daraufhin wird der Gefäßzugang zwischen arterieller und venöser Kanüle abgedrückt. Die Steuereinheit 18 erniedrigt nun, z. B. nach einer Bestätigung durch die Bedienperson den extrakorporalen Blutfluß ausgehend von dem oberen Grenzwert bis zu dem unteren Grenzwert, wobei der arterielle und venöse Druck $p_{art\ komp}$, $p_{ven\ komp}$ wieder gemessen und die Meßwerte gespeichert werden. Die Recheneinheit 27 liest die gespeicherten Meßwerte aus und berechnet aus den Meßwerten nach einem der oben beschriebenen Algorithmen den Fistelfluß $Q_F$ und den statischen arteriellen und venösen Druck in der Fistel. Fistelfluß und Fisteldruck werden dann auf der Anzeigeeinheit 28 angezeigt.

## Patentansprüche

1. Vorrichtung zur Ermittlung des Blutflusses in einem Gefäßzugang für eine extrakorporale Blutbehandlungsvorrichtung, bei der Blut über einen arteriellen Zweig eines extrakorporalen Kreislaufs, der an einem arteriellen Anschluss mit dem Gefäßzugang in Fluidverbindung steht, in die Blutbehandlungseinheit der Blutbehandlungsvorrichtung gelangt und über einen venösen Zweig des extrakorporalen Kreislaufs, der an einem venösen Anschluss mit dem Gefäßzugang in Fluidverbindung steht, zurückgeführt wird, wobei in den extrakorporalen Kreislauf eine Blutpumpe geschaltet ist, mit
einer Steuereinheit (18) zum Verändern der Flussrate der Blutpumpe,
einer arteriellen und/oder venösen Messeinrichtung (20, 22) zum Messen des Drucks $p_{art}$, $p_{ven}$ im arteriellen bzw. venösen Zweig (19, 21) des extrakorporalen Kreislaufs (2) bei offenem Gefäßzugang, während Blut durch denselben zwischen dem arteriellen und venösen Anschluss (12, 13) strömt, und zum Messen des Drucks $p_{art\ komp}$, $p_{ven\ komp}$ im arteriellen bzw. venösen Zweig (19, 21) bei unterbrochenem Gefäßzugang, während kein Blut durch denselben zwischen dem arteriellen und venösen Anschluss strömt, wobei der Blutfluss $Q_B$ im extrakorporalen Kreislauf zwischen den Messungen verändert wird,
einer Speichereinheit (25) zum Speichern der Werte des gemessenen arteriellen und/oder venösen Drucks, und einer Recheneinheit (27), die derart ausgebildet ist, dass aus mindestens zwei gemessenen Werten des arteriellen und/oder venösen Drucks $p_{art,}$ $p_{ven}$ bei offenem und mindestens zwei gemessenen Werten des arteriellen und/oder venösen Drucks $p_{art\ komp}$, $p_{ven\ komp}$ bei unterbrochenem Gefäßzugang der Blutfluss $Q_F$ im offenen Gefäßzugang zwischen dem arteriellen und venösen Anschluss bestimmbar ist.

2. Vorrichtung nach Anspruch 1, **dadurch gekennzeichnet, dass** die Steuereinheit (25) derart ausgebildet ist, dass in einem ersten Takt der Blutfluss im extrakorporalen Kreislauf innerhalb eines vorgegebenen Bereichs veränderbar ist, so dass mit der arteriellen und/oder venösen Messeinrichtung (20, 22) der Druck im arteriellen bzw. venösen Zweig bei offenem Gefäßzugang messbar und die Messwerte in der Speichereinheit (25) speicherbar sind, und in einem zweiten Takt der Blutfluss in dem vorgegebenen Bereich nochmals veränderbar ist, so dass mit der arteriellen und/oder venösen Messeinrichtung der Druck im arteriellen bzw. venösen Zweig bei unterbrochenem Gefäßzugang messbar und die Messwerte in der Speichereinheit speicherbar sind.

3. Vorrichtung nach Anspruch 1 oder 2, **dadurch gekennzeichnet, dass** die Recheneinheit (27) derart ausgebildet ist, dass zur Ermittlung des Blutflusses im offenen Gefäßzugang derjenige Blutfluss $Q_B$ im extrakorporalen Kreislauf bestimmbar ist, bei dem der Druck $p_{art\ komp}$ im arteriellen Zweig bei unterbrochenem Gefäßzugang gleich dem Druck $p_{art}$ im arteriellen Zweig bei offenem Gefäßzugang ist.

4. Vorrichtung nach Anspruch 1 oder 2, **dadurch gekennzeichnet, dass** die Recheneinheit (27) derart ausgebildet ist, dass zur Ermittlung des Blutflusses im offenen Gefäßzugang derjenige Blutfluss $Q_B$ im extrakorporalen Kreislauf bestimmbar ist, bei dem der Druck $P_{ven\ komp}$ im venösen Zweig bei unterbrochenem Gefäßzugang gleich dem Druck $p_{ven}$ im venösen Zweig bei offenem Gefäßzugang ist.

5. Vorrichtung nach Anspruch 1 oder 2, dass die Recheneinheit (27) derart ausgebildet ist, dass der Blutfluss im offenen Gefäßzugang aus dem ersten Blutfluss im extrakorporalen Kreislauf, bei dem der Druck $p_{art\ komp}$ im arteriellen Zweig bei unterbrochenem Gefäßzugang gleich dem Druck $p_{art}$ im arteriellen Zweig bei offenem Gefäßzugang ist, und dem zweiten Blutfluss im extrakorporalen Kreislauf bestimmbar ist, bei dem der Druck $p_{ven\ komp}$ im venösen Zweig bei unterbrochenem Gefäßzugang gleich dem Druck $p_{ven}$ im venösen Zweig bei offenem Gefäßzugang ist.

6. Vorrichtung nach Anspruch 5, **dadurch gekennzeichnet, dass** die Recheneinheit (27) derart ausgebildet ist, dass der Blutfluss im offenen Gefäßzugang aus dem Mittelwert des ermittelten ersten und zweiten Blutflusses bestimmbar ist.

7. Vorrichtung nach Anspruch 1 oder 2, **dadurch gekennzeichnet, dass** die Recheneinheit (27) derart ausgebildet ist, dass zur Ermittlung des Blutflusses im offenem Gefäßzugang derjenige extrakorporale Blutfluss $Q_B$ bestimmbar ist, bei dem die Differenz $\Delta p_{art}$ zwischen dem Druck $p_{art\ komp}$ im arteriellen Zweig bei unterbrochenem Gefäßzugang und dem Druck $p_{art}$ im arteriellen Zweig bei offenem Gefäßzugang gleich null ist und/oder die Differenz $\Delta p_{ven}$ zwischen dem Druck $p_{ven\ komp}$ im venösen Zweig bei unterbrochenem Gefäßzugang und dem Druck im venösen Zweig $p_{ven}$ bei offenem Gefäßzugang gleich null ist.

8. Vorrichtung nach einem der Ansprüche 1 bis 7, **dadurch gekennzeichnet, dass** die Recheneinheit (27) derart ausgebildet ist, dass zur Ermittlung des arteriellen und/oder venösen statischen Drucks im Gefäßzugang derjenige arterielle bzw. venöse Druck $p_{art}$, $p_{ven}$ im extrakorporalen Kreislauf bestimmbar ist, bei dem der Blutfluss im extrakorporalen Kreislauf gleich null ist.

9. Vorrichtung nach einem der Ansprüche 1 bis 8, **dadurch gekennzeichnet, dass** die Recheneinheit (27) derart ausgebildet ist, dass aus den Werten des arteriellen und/oder venösen Drucks $p_{art}$, $p_{ven}$, $P_{art\ komp}$, $p_{ven\ komp}$ im arteriellen und/oder venösen Zweig des extrakorporalen Kreislaufs bei unterbrochenem und offenem Gefäßzugang die Parameter einer den arteriellen bzw. venösen Druck in Abhängigkeit vom extrakorporalen Blutfluss $Q_B$ darstellenden Funktion $p(Q_B)$ bestimmbar sind.

**Claims**

1. A device for determining the blood flow in a vascular access for an extracorporeal blood treatment apparatus, wherein the blood passes via an arterial branch of an extracorporeal circuit, which is in fluid connection with an arterial connection to the vascular access, into the blood treatment unit of the blood treatment apparatus and is fed back via a venous branch of the extracorporeal circuit, which is in fluid connection with a venous connection, to the vascular access, whereby a blood pump is incorporated into the extracorporeal circuit, with

a control unit (18) for changing the flow rate of the blood pump,
an arterial and/or venous measuring device (20, 22) for measuring pressure $p_{art}$, $p_{ven}$ in the arterial and venous branch (19, 21) of the extracorporeal circuit (2) with open vascular access, whilst blood is flowing through the same between the arterial and venous connection (12, 13), and for measuring pressure $P_{art\,comp}$, $p_{ven\,comp}$ in the arterial and venous branch (19, 21) with interrupted vascular access, whilst no blood is flowing through the same between the arterial and venous connection, whereby blood flow $Q_B$ in the extracorporeal circuit is changed between measurements,
a memory unit (25) for storing the values of the measured arterial and/or venous pressure, and
a computer unit (27), which is designed in such a way that blood flow $Q_F$ in the open vascular access between the arterial and venous connection can be determined from at least two measured values of the arterial and/or venous pressure $p_{art}$, $p_{ven}$ with open vascular access and at least two measured values of the arterial and/or venous pressure $p_{art\,comp}$, $p_{ven\,comp}$ with interrupted vascular access.

2. The device according to claim 1, **characterised in that** the control unit (25) is designed in such a way that, in a first cycle, the blood flow in the extracorporeal circuit can be changed inside a predetermined region, so that the pressure in the arterial and venous branch with open vascular access can be measured with the arterial and/or venous measuring device (20, 22) and the measured values can be stored in the memory unit (25), and in a second cycle the blood flow can again be changed in the predetermined region, so that the pressure in the arterial or venous branch with interrupted vascular access can be measured with the arterial and/or venous measuring device and the measured values can be stored in the memory unit.

3. The device according to claim 1 or 2, **characterised in that** the computer unit (27) is designed in such a way that, in order to determine the blood flow in the open vascular access, it is possible to determine blood flow $Q_B$ in the extracorporeal circuit with which pressure $p_{art\,comp}$ in the arterial branch with interrupted vascular access is equal to pressure $p_{art}$ in the arterial branch with open vascular access.

4. The device according to claim 1 or 2, **characterised in that** the computer unit (27) is designed in such a way that, in order to determine the blood flow in the open vascular access, it is possible to determine blood flow $Q_B$ in the extracorporeal circuit with which pressure $p_{ven\,comp}$ in the venous branch with interrupted vascular access is equal to pressure $p_{ven}$ in the venous branch with open vascular access.

5. The device according to claim 1 or 2, **characterised in that** the computer unit (27) is designed in such a way that the blood flow in the open vascular access can be determined from the first blood flow in the extracorporeal circuit, with which pressure $p_{art\,comp}$ in the arterial branch with interrupted vascular access is equal to pressure $p_{art}$ in the arterial branch with open vascular access, and the second blood flow in the extracorporeal circuit, with which pressure $p_{ven\,comp}$ in the venous branch with interrupted vascular access is equal to pressure $p_{ven}$ in the venous branch with open vascular access.

6. The device according to claim 5, **characterised in that** the computer unit (27) is designed in such a way that the blood flow in the open vascular access can be determined from the mean value of the ascertained first and second blood flow.

7. The device according to claim 1 or 2, **characterised in that** the computer unit (27) is designed in such a way that, in order to determine the blood flow in the open vascular access, it is possible to determine extracorporeal blood flow $Q_B$ with which difference $\Delta p_{art}$ between pressure $p_{art\,comp}$ in the arterial branch with interrupted vascular access and pressure $p_{art}$ in the arterial branch with open vascular access is equal to zero and/or difference $\Delta p_{ven}$ between pressure $p_{ven\,comp}$ in the venous branch with interrupted vascular access and the pressure in the venous branch $p_{ven}$ with open vascular access is equal to zero.

8. The device according to any one of claims 1 to 7, **characterised in that** the computer unit (27) is designed in such a way that, in order to determine the arterial and/or venous static pressure in the vascular access, it is possible to determine the arterial or venous pressure $p_{art}$, $p_{ven}$ in the extracorporeal circuit with which the blood flow in the extracorporeal circuit is equal to zero.

9. The device according to any one of claims 1 to 8, **<u>characterised in that</u>** the computer unit (27) is designed in such a way that the parameters of a function p ($Q_B$) representing the arterial and venous pressure as a function of extracorporeal blood flow $Q_B$ can be determined from the values of the arterial and/or venous pressure $p_{art}$, $p_{ven}$, $p_{art\ comp}$, $p_{ven\ comp}$ in the arterial and/or venous branch of the extracorporeal circuit with interrupted and open vascular access.

## Revendications

1. Dispositif permettant de déterminer le flux sanguin dans un accès à un vaisseau, pour un dispositif extracorporel de traitement du sang dans lequel du sang, via une branche artérielle d'une circulation extracorporelle qui est en communication fluidique avec l'accès à un vaisseau, au niveau d'un raccord artériel, parvient dans l'unité de traitement du sang du dispositif de traitement du sang et, via une branche veineuse de la circulation extracorporelle qui est en communication fluidique avec l'accès à un vaisseau, au niveau d'un raccord veineux, est renvoyé, où une pompe à sang est montée dans la circulation extracorporelle, dispositif comprenant :

   - une unité de commande (18) servant à modifier le débit de la pompe à sang,
   - un dispositif de mesure artériel et/ou veineux (20, 22) servant à mesurer la pression $P_{art}$, $P_{ven}$ dans la branche artérielle ou veineuse (19, 21) de la circulation extracorporelle (2), lorsqu'un accès à un vaisseau est ouvert, tandis que du sang circule à travers ce même accès à un vaisseau entre le raccord artériel et veineux (12, 13), et servant à mesurer la pression $p_{art\ komp}$, $p_{ven\ komp}$ dans la branche artérielle ou veineuse (19, 21), lorsqu'un accès à un vaisseau est interrompu, tandis qu'aucune quantité de sang ne circule à travers ce même accès à un vaisseau entre le raccord artériel et veineux, où le flux sanguin $Q_B$ de la circulation extracorporelle est modifié entre les mesures,
   - une unité de mémoire (25) servant à mémoriser les valeurs de la pression artérielle et/ou veineuse mesurée, et
   - une unité de calcul (27) qui est configurée de manière telle, que le flux sanguin $Q_F$ puisse être déterminé dans l'accès ouvert à un vaisseau, entre le raccord artériel et veineux, à partir d'au moins deux valeurs mesurées de la pression artérielle et/ou veineuse $p_{art}$, $p_{ven}$, lorsque l'accès à un vaisseau est ouvert, et à partir d'au moins deux valeurs mesurées de la pression artérielle et/ou veineuse $p_{art\ komp}$, $p_{ven\ komp}$, lorsque l'accès à un vaisseau est interrompu.

2. Dispositif selon la revendication 1, **caractérisé en ce que** l'unité de commande (18) est conçue de manière telle, que dans un premier cycle, le flux sanguin de la circulation extracorporelle soit modifiable à l'intérieur d'une plage prédéterminée, de sorte qu'avec le dispositif de mesure artériel et/ou veineux (20, 22), la pression soit mesurable dans la branche artérielle ou veineuse, lorsque l'accès à un vaisseau est ouvert, et que les valeurs mesurées soient mémorisables dans l'unité de mémoire (25), et que, dans un deuxième cycle, le flux sanguin soit encore modifiable dans la plage prédéterminée, de sorte qu'avec le dispositif de mesure artériel et/ou veineux, la pression dans la branche artérielle ou veineuse soit mesurable, lorsque l'accès à un vaisseau est interrompu, et que les valeurs mesurées soient mémorisables dans l'unité de mémoire.

3. Dispositif selon la revendication 1 ou 2, **caractérisé en ce que** l'unité de calcul (27) est conçue de manière telle, que pour le calcul du flux sanguin, lorsque l'accès à un vaisseau est ouvert, l'on puisse déterminer le flux sanguin $Q_B$ de la circulation extracorporelle, dans lequel la pression $p_{art\ komp}$ dans la branche artérielle, lorsque l'accès à un vaisseau est interrompu, est égale à la pression $P_{art}$ dans la branche artérielle, lorsque l'accès à un vaisseau est ouvert.

4. Dispositif selon la revendication 1 ou 2, **caractérisé en ce que** l'unité de calcul (27) est conçue de manière telle, que pour le calcul du flux sanguin, lorsque l'accès à un vaisseau est ouvert, l'on puisse déterminer le flux sanguin $Q_B$ de la circulation extracorporelle, dans lequel la pression $p_{ven\ komp}$ dans la branche veineuse, lorsque l'accès à un vaisseau est interrompu, est égale à la pression $p_{ven}$ dans la branche veineuse, lorsque l'accès à un vaisseau est ouvert.

5. Dispositif selon la revendication 1 ou 2, **caractérisé en ce que** l'unité de calcul (27) est conçue de manière telle, que lorsque l'accès à un vaisseau est ouvert, l'on puisse déterminer le flux sanguin provenant du premier flux sanguin de la circulation extracorporelle, dans lequel la pression $p_{art\ komp}$ dans la branche artérielle, lorsque l'accès à un vaisseau est interrompu, est égale à la pression $p_{art}$ dans la branche artérielle, lorsque l'accès à un vaisseau est ouvert, et le flux sanguin provenant du deuxième flux sanguin de la circulation extracorporelle, dans lequel la pression $p_{ven\ komp}$ dans la branche veineuse, lorsque l'accès à un vaisseau est interrompu, est égale à la pression

$p_{ven}$ dans la branche veineuse, lorsque l'accès à un vaisseau est ouvert.

6. Dispositif selon la revendication 5, **caractérisé en ce que** l'unité de calcul (27) est conçue de manière telle, que le flux sanguin, lorsque l'accès à un vaisseau est ouvert, puisse être déterminé à partir de la valeur moyenne des premier et deuxième flux sanguins calculés.

7. Dispositif selon la revendication 1 ou 2, **caractérisé en ce que** l'unité de calcul (27) est conçue de manière telle, que pour le calcul du flux sanguin, lorsque l'accès à un vaisseau est ouvert, l'on puisse déterminer le flux sanguin extracorporel $Q_B$ dans lequel la différence $\Delta p_{art}$ entre la pression $P_{art\,komp}$ dans la branche artérielle, lorsque l'accès à un vaisseau est interrompu, et la pression $p_{art}$ dans la branche artérielle, lorsque l'accès à un vaisseau est ouvert, est égale à zéro, et/ou la différence $\Delta p_{ven}$ entre la pression $p_{ven\,komp}$ dans la branche veineuse, lorsque l'accès à un vaisseau est interrompu, et la pression $p_{ven}$ dans la branche veineuse, lorsque l'accès à un vaisseau est ouvert, est égale à zéro.

8. Dispositif selon l'une quelconque des revendications 1 à 7, **caractérisé en ce que** l'unité de calcul (27) est conçue de manière telle, que pour le calcul de la pression statique artérielle et/ou veineuse, au niveau de l'accès à un vaisseau, l'on puisse déterminer la pression artérielle ou veineuse $p_{art}$, $p_{ven}$ de la circulation extracorporelle, pression dans le cas de laquelle le flux sanguin de la circulation extracorporelle est égal à zéro.

9. Dispositif selon l'une quelconque des revendications 1 à 8, **caractérisé en ce que** l'unité de calcul (27) est conçue de manière telle, qu'à partir des valeurs de la pression artérielle et/ou veineuse $P_{art}$, $P_{ven}$, $P_{art\,komp}$, $p_{ven\,komp}$ dans la branche artérielle et/ou veineuse de la circulation extracorporelle, lorsque l'accès à un vaisseau est interrompu et ouvert, l'on puisse déterminer les paramètres d'une fonction $p(Q_B)$ représentant la pression artérielle ou veineuse, en fonction du flux sanguin extracorporel $Q_B$.

Fig. 1

$$Q_F - Q_B$$

**Fig. 2**

**Fig. 3**

Fig. 4

Fig. 5